(19) Europäisches Patentamt
European Patent Office
Office européen des brevets

(11) EP 0 664 278 B1

(12) **FASCICULE DE BREVET EUROPEEN**

(45) Date de publication et mention de la délivrance du brevet:
**06.08.1997 Bulletin 1997/32**

(51) Int. Cl.[6]: **C07C 5/27**, B01J 21/06

(21) Numéro de dépôt: **94400158.5**

(22) Date de dépôt: **25.01.1994**

(54) **Procédé pour l'isomérisation des oléfines**
Verfahren zur Isomerisierung von Olefinen
Process for the isomerisation of olefins

(84) Etats contractants désignés:
**BE ES NL**

(43) Date de publication de la demande:
**26.07.1995 Bulletin 1995/30**

(73) Titulaire: **INSTITUT FRANCAIS DU PETROLE**
**92502 Rueil-Malmaison (FR)**

(72) Inventeurs:
- **Travers, Christine**
  **F-92500 Rueil Malmaison (FR)**
- **Burzynski, Jean-Pierre**
  **F-69110 Sainte-Foy-les-Lyon (FR)**
- **Dagand, Jean**
  **F-75011 Paris (FR)**
- **Courty, Philippe**
  **F-78800 Houilles (FR)**

(74) Mandataire: **Andreeff, François**
**INSTITUT FRANCAIS DU PETROLE**
**4, avenue de Bois-Préau**
**92502 Rueil-Malmaison (FR)**

(56) Documents cités:
**EP-A- 0 588 680** **US-A- 4 814 519**
- **DATABASE WPI Week 8434, Derwent Publications Ltd., London, GB; AN 84-210999 & JP-A-59 123 538 (MITSUBISHI PETROCH) 17 Juillet 1984**

EP 0 664 278 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention décrit un procédé d'isomérisation des oléfines présentant au plus 20 atomes de carbone et plus particulièrement d'isomérisation des n-butènes en isobutènes et des n-pentènes en isopentènes (iso amylènes), utilisant un catalyseur particulier.

La réduction des alkyls de plomb dans les essences a conduit le raffineur à envisager depuis quelques années, l'incorporation à l'essence de différents composés et en particulier, des alcools et des esters, permettant d'augmenter l'indice d'octane. Outre le méthanol qui est l'un des plus intéressants additifs connu, le MTBE (méthyl-tertio-butyl éther), possède des propriétés antidétonantes permettant l'amélioration de la qualité des essences et l'augmentation de leur indice d'octane, augmentation supérieure à celle obtenue avec le méthanol. Le MTBE possède également de nombreux autres avantages tels que :

- un point d'ébullition correspondant à celui des composants de l'essence qui présentent les plus faibles propriétés antidétonantes,
- une tension de vapeur compatible avec les composants précités,
- un excellent point de congélation,
- une solubilité dans l'eau faible,
- une miscibilité complète avec les hydrocarbures etc...

Le MTBE est généralement obtenu à partir d'isobutène et de méthanol d'après la réaction suivante :

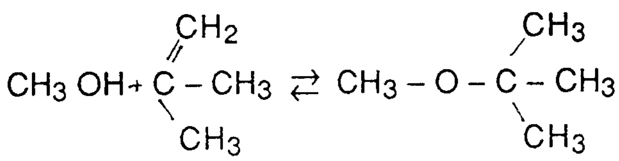

L'isobutène est généralement contenu dans les coupes oléfiniques $C_3$-$C_4$, issues des effluents du craquage catalytique, du vapocraquage, du craquage thermique et du procédé de réduction de viscosité. Cependant les quantités d'isobutène fournies par ces différents procédés ne sont pas suffisantes pour permettre un large développement du procédé de production de MTBE.

C'est pourquoi, il a été proposé, afin de produire de plus grandes quantités d'isobutène, d'isomériser complètement ou presque, les butènes contenus dans les effluents des procédés cités ci-dessus en isobutènes.

De nombreux procédés associés à de nombreux catalyseurs ont été proposés dans la littérature. Les catalyseurs utilisés sont généralement à base d'alumine, ou plus particulièrement d'alumines activées ou traitées à la vapeur (US 3 558 733), qu'il s'agisse d'alumine $\eta$ ou $\gamma$, d'alumines halogénées, de bauxite, d'alumines traitées avec des dérivés du bore, du silicium (US 4 013 590, US 4 038 337, GB 2 129 701 et US 4 434 315) ou du zirconium et diverses silice-alumines etc...

La plupart de ces catalyseurs présente une conversion par passe relativement faible et une faible sélectivité due aux réactions parasites telles que le craquage et la polymérisation, ces dernières entraînant de plus une rapide décroissance des performances.

## OBJET DE L'INVENTION

La présente invention consiste à utiliser un catalyseur permettant d'obtenir des performances améliorées, en particulier au niveau de la sélectivité et présentant une stabilité accrue.

Il a été découvert qu'un catalyseur obtenu à partir d'alumine et de préférence d'alumine éta ou gamma, à laquelle on a ajouté une quantité bien déterminée de titane (0,03 à 0,6 % en poids) et auquel on fait subir de préférence un traitement à la vapeur d'eau dans des conditions précises, conduisait de manière surprenante, à des sélectivités, conversions et durée de cycle très nettement améliorées.

## DISCUSSION DÉTAILLÉE

Dans le procédé suivant l'invention, il est possible d'isomériser soit une coupe $C_4$ oléfinique seule issue des procédés précédemment cités, après avoir enlevé la coupe $C_3$, soit la totalité de la coupe $C_3$-$C_4$ oléfinique.

Selon l'invention, des hydrocarbures oléfiniques linéaires contenant de 5 à 20 atomes de carbone par molécules,

peuvent être également isomérisés.

La charge à isomériser est mise au contact du catalyseur à une température comprise entre 300 °C et 570 °C (et de préférence entre 400 et 550 °C lorsqu'elle est constituée de butènes et/ou de pentènes) à une pression comprise entre 1 et 10 bars absolus (et préférentiellement entre 1 et 5 bars absolus lorsque la charge est constituée de butènes et/ou de pentènes).

La vitesse spatiale est comprise entre 0,1 et 10 $h^{-1}$ exprimée en volume de charge oléfinique par volume de catalyseur et par heure (et de manière préférée entre 0,5 et 6 $h^{-1}$ lorsque la charge est constituée de butènes et/ou de pentènes).

Selon l'invention, le procédé est mis en oeuvre en présence d"eau, afin de minimiser les réactions secondaires indésirables. La quantité d'eau introduite dans le réacteur est telle que le rapport molaire $H_2O$/hydrocarbures oléfiniques soit compris entre 0,1 et 10 (et préférentiellement entre 0,5 et 3 lorsque la charge est constituée de butènes et/ou de pentènes).

Pour la préparation du catalyseur, on peut utiliser des alumines commerciales, de préférence activées, choisies de manière préférée dans le groupe des alumines éta et gamma, et ayant une faible teneur en alcalins, par exemple contenant moins de 0,1 % de sodium.

La surface spécifique de l'alumine sera avantageusement comprise entre 10 et 500 $m^2/g$ et de préférence entre 50 et 450 $m^2/g$, son volume poreux étant compris entre 0,4 et 0,8 $cm^3/g$.

Les catalyseurs selon l'invention, sont préparés en ajoutant de 0,05 à 1 % et de préférence de 0,085 à 0,5 % de bioxyde de titane sur le support d'alumine. Tout procédé permettant l'ajout de ce bioxyde de titane peut convenir. On peut, par exemple, dissoudre un composé de titane dans la solution contenant le composé d'aluminium et ajuster les conditions de précipitation de l'alumine pour que l'hydroxyde de titane coprécipite. On peut également ajouter à l'alumine hydratée sous forme de gel ($\alpha$-trihydrate, $\beta$-trihydrate ou $\alpha$-monohydrate d'aluminium) au moins un composé de titane choisi dans le groupe formé par le bioxyde de titane sous les formes rutile et anatase, les sous-oxydes TiO et $Ti_2O_3$, les acides titaniques, les titanates alcalins, alcalinoterreux et d'ammonium et les sels solubles et insolubles, organiques et inorganiques du titane.

On peut également partir d'un support d'alumine mis en forme et l'imprégner avec une solution d'un sel organique ou inorganique de titane ; d'une façon générale, l'addition de titane peut être conduite avant la mise en forme, au cours de la mise en forme ou après mise en forme du support de catalyseur.

Un procédé préféré consiste à ajouter à une solution organique (par exemple alcoolique) d'au moins un composé organique d'aluminium (par exemple un alcoxyaluminium tel que l'isopropylate d'aluminium), au moins un composé organique du titane, par exemple le tétraéthoxytitane, puis à hydrolyser la solution ainsi obtenue.

On peut également ajouter le titane sous forme d'un composé inorganique facilement hydrolysable tel que le tétrachlorure de titane $TiCl_4$.

Un autre procédé préféré consiste à ajouter en quantités contrôlées un composé organique à base de titane, par exemple un alcoxytitane tel que le tétraéthyltitane et/ou un composé inorganique du titane (par exemple le trichlorure de titane) au cours de la synthèse ZIEGLER du polyalcoxyaluminium, par réaction d'un alkylaluminium, (par exemple le triéthylaluminium), de l'éthylène, et d'au moins un composé précité du titane. Par polymérisation puis oxydation subséquente, on prépare le polyalcoxyaluminium précité, dont l'hydrolyse conduira aux polyols et à l'alumine hydratée contenant du titane.

On a constaté expérimentalement que ces procédés conduisaient à une dispersion particulièrement élevée des ions titane dans la matrice d'alumine, obtenue après hydrolyse de l'alcoxyaluminium ou du polyalcoxyaluminium. Les procédés préférés d'imprégnation du titane, permettent, par exemple, lorsque le support se présente sous forme de billes ou d'extrudés etc..., d'obtenir une teneur en $TiO_2$ constante d'une bille à l'autre ou d'un extrudé à l'autre ; si la concentration moyenne désirée est C %, la concentration, C, d'une bille à l'autre ou d'un extrudé à l'autre restera, avec les méthodes préférées de l'invention comprise entre C $\pm$ 5 % de cette concentration et même comprise entre $\pm$ 3 % en poids. Des résultats encore améliorés ont été obtenus en utilisant des supports de catalyseur contenant plus particulièrement de 0,06 à 0,15 % $TiO_2$.

La teneur en titane sur le catalyseur est mesurée par fluorescence X.

Le catalyseur ainsi obtenu est ensuite séché à une température comprise entre 100 et 130 °C et éventuellement calciné sous air à une température comprise entre 400 et 800 °C et de préférence entre 450 et 750 °C pendant des temps variant de 1 à 5 heures. Il peut ensuite être avantageusement traité sous vapeur d'eau à une température comprise entre 120 et 700 °C et de manière préférée entre 300 et 700 °C sous une pression partielle de vapeur d'eau supérieure à 0,5 bars et de préférence comprise entre 0,6 et 1 bars pendant une durée de 0,5 à 120 heures et de préférence de 1 h à 100 h.

Les performances en isomérisation seront exprimées par

1/ la conversion des butènes

$$C = \frac{\Sigma\text{ (\% n-butènes) charge - }\Sigma\text{ (\% n-butènes) effluent}}{\Sigma\text{ (\% n-butènes) charge}} \times 100$$

2/ la sélectivité en isobutènes

$$S = \frac{\text{(\% isobutène) effluent - (\% isobutène) charge}}{\Sigma\text{ (\% n-butènes) charge - }\Sigma\text{ (\% n-butènes effluent)}} \times 100$$

3/ le rendement en isobutène

$$R = C \times S/100$$

Les exemples qui suivent précisent l'invention sans en limiter la portée.

**EXEMPLE 1 - Catalyseur A non conforme à l'invention**

Un support d'alumine $\gamma$ commercial, de surface 200 $m^2/g$ est soumis à un traitement à la vapeur d'eau à 560 °C pendant 20 heures à raison avec une pression partielle de vapeur d'eau égale à 0,8 bars. Ce catalyseur est mis en oeuvre en isomérisation d'une coupe $C_4$, oléfinique dont la composition figure tableau I. Les conditions opératoires sont les suivantes :

LHSV = 2 $h^{-1}$
$H_20/C_4$= (mole) = 2
T = 530°C
p = 1 bar absolu

Les performances obtenues figurent tableau I après 1 heure de fonctionnement et tableau II après 30 heures de fonctionnement.

On peut voir que les performances sont faibles et qu'elles diminuent au cours du temps.

**EXEMPLE 2 - Catalyseur B conforme à l'invention**

Le support d'alumine $\gamma$ commercial utilisé dans l'exemple 1 et imprégné de 0,1 % de titane à partir d'oxalate de titane décahydraté en solution aqueuse, puis séché à 100°C pendant 2 heures et calciné à 600 °C pendant 2 heures. Le catalyseur B ainsi obtenu est soumis à un traitement sous vapeur d'eau, équivalent à celui décrit dans l'exemple 1, puis mis en oeuvre dans le procédé d'isomérisation d'une coupe $C_4$ oléfinique, dans les conditions opératoires décrites ci-dessus.

Les performances obtenues figurent tableau I après 1 heure de fonctionnement et tableau II après 30 heures de fonctionnement.

Les performances obtenues avec le catalyseur B selon l'invention sont nettement supérieures à celles obtenues avec le catalyseur A non conforme en activité, sélectivité et stabilité.

**EXEMPLE 3 - Catalyseur C conforme à l'invention**

Le catalyseur C diffère du catalyseur B, en ce qu'il n'est pas soumis à un traitement sous vapeur d'eau après dépôt de Ti, séchage et calcination effectués dans les conditions opératoires décrites dans l'exemple 2.

Le catalyseur C est mis en oeuvre dans le procédé d'isomérisation d'une coupe $C_4$ oléfinique, dans les conditions opératoires décrites ci-dessus.

Les performances obtenues avec le catalyseur C figurent tableau I après 1 heure de fonctionnement et tableau II après 30 heures de fonctionnement.

On observe que les performances du catalyseurs C se situent entre celles des catalyseurs A et B.

Dans le tableau I, $C_2$ désigne l'éthane, $C_{2=}$ : l'éthylène, $C_3$ : le propane, $iC_4$ : l'isobutane, $nC_4$ : le n-butane, $C_4$=2TR : le butène-2trans, $C_4$=1 : le butène-1, $iC_{4=}$ : l'isobutène, $C_4$=2Cis : le butène-2cis, $C_4$== : le butadiène et $C_{5+}$ : les hydrocarbures à plus de 5 atomes de carbone.

Tableau I

| $H_20/C_4$= (mole) | - | 2 | 2 | 2 |
|---|---|---|---|---|
| LHSV ($h^{-1}$) | - | 2 | 2 | 2 |
| Temps de fonctionnement (h) | - | 1 | 1 | 1 |
| | **Charge** | **Catalyseur A** | **Catalyseur B** | **Catalyseur C** |
| $CH_4$ | 0 | 0,052 | 0,06 | 0,03 |
| $C_2$ | 0 | 0,016 | 0,018 | 0,01 |
| $C_2$= | 0 | 0,126 | 0,315 | 0,12 |
| $C_3$ | 0 | 0,005 | 0 | 0 |
| $C_3$= | 0 | 1,228 | 2,571 | 1,52 |
| $iC_4$ | 0,216 | 0,28 | 0,292 | 0,29 |
| $nC_4$ | 21,76 | 21,537 | 21,545 | 21,55 |
| $C_4$=2TR | 2,732 | 22,44 | 18,5 | 21,1 |
| $C_4$=1 | 63,878 | 16,37 | 14,8 | 15,3 |
| $iC_4$= | 5,617 | 19,11 | 25,73 | 22,13 |
| $C_4$=2Cis | 5,139 | 17,38 | 14,25 | 16,35 |
| $C_4$== | 0,03 | 0,088 | 0,07 | 0,08 |
| $C_5$+ | 0,628 | 1,368 | 1,85 | 1,52 |
| Conversion % | - | 22,25 | 33,7 | 26,5 |
| Sélectivité % | - | 83,7 | 83,1 | 86,8 |
| Rendement % | | 18,6 | 28,0 | 23,0 |

Tableau II

| | **Catalyseur A** | **Catalyseur B** | **Catalyseur C** |
|---|---|---|---|
| $H_20/C_4$= (mole) | 2 | 2 | 2 |
| LHSV ($h^{-1}$) | 2 | 2 | 2 |
| Temps de fonctionnement (h) | 30 | 30 | 30 |
| Conversion (%) | 21,0 | 33,45 | 25,5 |
| Sélectivité (%) | 86,0 | 83,5 | 87,5 |
| Rendement (%) | 18,05 | 27,9 | 22,0 |

## Revendications

1. Procédé d'isomérisation des hydrocarbures oléfiniques linéaires ayant au plus 20 atomes de carbone par molécule, caractérisé en ce que lesdits hydrocarbures sont mis au contact d'un catalyseur contenant de l'alumine et 0,03 à 0,6 % en poids de titane, à une température comprise entre 300 et 570 °C, une pression comprise entre 0,1 et 1 MPa, une vitesse spatiale comprise entre 0,1 et 10 $h^{-1}$, et en présence d'injection d'eau, le rapport molaire eau injectée/hydrocarbures oléfiniques étant compris entre 0,1 et 10.

2. Procédé selon la revendication 1 caractérisé en ce que le catalyseur contenant l'alumine et le titane a subi un traitement à la vapeur d'eau avant d'être mis au contact des hydrocarbures, ledit traitement ayant lieu entre 120 et 700 °C sous une pression partielle de vapeur d'eau supérieure à 0,5 bars pendant une durée de 0,5 à 120 h.

3. Procédé selon l'une des revendications précédentes, caractérisé en ce que les hydrocarbures oléfiniques linéaires traités sont choisis dans le groupe formé par les butènes et les pentènes.

4. Procédé selon l'une des revendications précédentes, caractérisé en ce que la température d'isomérisation est comprise entre 400 et 550°C.

5. Procédé selon l'une des revendications précédentes, caractérisé en ce que la pression d'isomérisation est comprise entre 0,1 et 0,5 MPa.

6. Procédé selon l'une des revendications précédentes, caractérisé en ce que la vitesse spatiale est comprise entre 0,5 et $6h^{-1}$.

7. Procédé selon la revendication 3, caractérisé en ce que le rapport molaire eau injectée/hydrocarbures est compris entre 0,5 et 3.

8. Procédé selon l'une des revendications précédentes, caractérisé en ce que l'alumine est une alumine gamma.

9. Procédé selon l'une des revendications précédentes, caractérisé en ce que l'alumine est une alumine éta.

10. Procédé selon l'une des revendications précédentes, caractérisé en ce que la teneur en sodium de l'alumine est inférieure à 0,1 % en poids.

11. Procédé selon l'une des revendications précédentes, caractérisé en ce que la surface spécifique de l'alumine est comprise entre 10 et 500 $m^2/g$.

12. Procédé selon l'une des revendications précédentes, caractérisé en ce que le volume poreux de l'alumine est compris entre 0,4 et 0,8 $cm^3/g$.

13. Procédé selon la revendication 2, caractérisé en ce que le traitement à la vapeur d'eau a lieu à une température de 300 à 700°C.

14. Procédé selon la revendication 2, caractérisé en ce que le traitement à la vapeur d'eau a lieu sous une pression partielle de vapeur d'eau comprise entre 0,6 et 1 bars.

**Claims**

1. A process for the isomerisation of straight chain olefinic hydrocarbons having at most 20 carbon atoms per molecule, characterised in that said hydrocarbons are brought into contact with a catalyst containing alumina and from 0.03 to 0.6% by weight of titanium, at a temperature of between 300 and 570°C, a pressure of between 0.1 and 1 MPa, at a space velocity of between 0.1 and 10 $h^{-1}$, and in the presence of water injection, the injected water/olefinic hydrocarbons molar ratio being between 0.1 and 10.

2. A process according to claim 1 characterised in that the catalyst containing alumina and titanium was subjected to a steam treatment before being brought into contact with the hydrocarbons, said treatment taking place at between 120 and 700°C under a steam partial pressure of higher than 0.5 bars for a period of time of from 0.5 to 120 hours.

3. A process according to one of the preceding claims characterised in that the straight chain olefinic hydrocarbons treated are selected fro the group formed by butenes and pentenes.

4. A process according to one of the preceding claims characterised in that the isomerisation temperature is between 400 and 550°C.

5. A process according to one of the preceding claims characterised in that the isomerisation pressure is between 0.1 and 0.5 MPa.

6. A process according to one of the preceding claims characterised in that the space velocity is between 0.5 and 6 $h^{-1}$.

7. A process according to claim 3 characterised in that the injected water/hydrocarbons molar ratio is between 0.5 and 3.

8. A process according to one of the preceding claims characterised in that the alumina is a gamma alumina.

9. A process according to one of the preceding claims characterised in that the alumina is an eta alumina.

10. A process according to one of the preceding claims characterised in that the amount of sodium in the alumina is less than 0.1% by weight.

11. A process according to one of the preceding claims characterised in that the specific surfaced area of the alumina is between 10 and 500$m^2$/g.

12. A process according to one of the preceding claims characterised in that the pore volume of the alumina is between 0.4 and 0.8 $cm^3$/g.

13. A process according to claim 2 characterised in that the stem treatment takes place at a temperature of from 300 to 700°C.

14. A process according to claim 2 characterised in that the stem treatment takes place at a stem partial pressure of between 0.6 and 1 bar.

**Patentansprüche**

1. Verfahren zur Isomerisierung von linearen olefinischen Kohlenwasserstoffen mit höchstens 20 Kohlenstoffatomen pro Molekül, dadurch gekennzeichnet, daß die Kohlenwasserstoffe mit einem Katalysator, der Aluminiumoxid und 0,03 bis 0,6 Gew. % Titan enthält, bei einer Temperatur, die zwischen 300 und 570°C liegt, einem Druck, der zwischen 0,1 und 1 MPa liegt, einer Raumgeschwindigkeit, die zwischen 0,1 und 10 $h^{-1}$ liegt und in Gegenwart einer Wasserinjektion in Kontakt gebracht werden, wobei das molare Verhältnis injiziertes Wasser/olefinische Kohlenwasserstoffe zwischen 0,1 und 10 liegt.

2. Verfahren nach Anspruch 1, dadurch gekennzeichnet, daß der Katalysator, der Aluminiumoxid und Titan enthält, vor dem in Kontakt bringen mit den Kohlenwasserstoffen einer Behandlung mit Wasserdampf unterworfen war, wobei die Behandlung zwischen 120 und 700°C unter einem Partialdruck von Wasserdampf über 0,5 bar während einer Dauer von 0,5 bis 120 Stunden stattfindet.

3. Verfahren nach einem der vorhergehenden Ansprüche, dadurch gekennzeichnet, daR die behandelten, linearen, olefinischen Kohlenwasserstoffe aus der durch die Butene und die Pentene gebildeten Gruppe ausgewählt sind.

4. Verfahren nach einem der vorhergehenden Ansprüche, dadurch gekennzeichnet, daß die Isomerisierungstemperatur zwischen 400 und 550°C enthalten ist.

5. Verfahren nach einem der vorhergehenden Ansprüche, dadurch gekennzeichnet, daß der Isomerisierungsdruck zwischen 0,1 und 0,5 MPa enthalten ist.

6. Verfahren nach einem der vorhergehenden Ansprüche, dadurch gekennzeichnet, daß die Raumgeschwindigkeit zwischen 0,5 und 6 $h^{-1}$ enthalten ist.

7. Verfahren nach Anspruch 3, dadurch gekennzeichnet, daß das Molverhältnis injiziertes Wasser/Kohlenwasserstoffe zwischen 0,5 und 3 liegt.

8. Verfahren nach einem der vorhergehenden Ansprüche, dadurch gekennzeichnet, daß das Aluminiumoxid $\gamma$-Aluminiumoxid ist.

9. Verfahren nach einem der vorhergehenden Ansprüche, dadurch gekennzeichnet, daß das Aluminiumoxid $\varepsilon$-Aluminiumoxid ist.

10. Verfahren nach einem der vorhergehenden Ansprüche, dadurch gekennzeichnet, daß der Natriumgehalt des Aluminiumoxides unter 0,1 Gew.% beträgt.

11. Verfahren nach einem der vorhergehenden Ansprüche, dadurch gekennzeichnet, daß die spezifische Oberfläche des Aluminiumoxides zwischen 10 und 500 $m^2/g$ beträgt.

12. Verfahren nach einem der vorhergehenden Ansprüche, dadurch gekennzeichnet, daß das Porenvolumen des Aluminiumoxides zwischen 0,4 und 0,8 $cm^3/g$ beträgt.

13. Verfahren nach Anspruch 2, dadurch gekennzeichnet, daß die Behandlung mit Wasserdampf bei einer Temperatur von 300 bis 700°C stattfindet.

14. Verfahren nach Anspruch 2, dadurch gekennzeichnet, daß die Behandlung mit Wasserdampf unter einem Partialdruck von Wasserdampf, der zwischen 0,6 und 1 bar liegt, stattfindet.